# EUROPEAN PATENT APPLICATION

(11) **EP 3 847 893 A1**
(43) Date of publication of application: **14.07.2021**
(21) Application number: 20151299.3
(22) Date of filing: 10.01.2020
(51) Int. Cl.: A23B 7/04, A23B 7/045, A23F 3/34, A23L 3/36, A23L 3/365, A61K 36/185, A23L 2/56

(54) **METHOD FOR PROCESSING A PLANT HAVING A STEM AND PRODUCT OBTAINED BY THE METHOD**

(71) Applicant: Tellement Facile SA, 1266 Duillier (CH)
(72) Inventor: MARAKHOV, Kostiantyn, 1266 Duillier (CH)
(74) Representative: Bird & Bird LLP

(57) **Abstract**

Method for processing a plant having a stem, comprising the following steps:
S3
freezing the plant to a second temperature (T2) below -10°C,
S6
preparing an infusion using the frozen plant and a liquid.

## Description

The present invention relates to a method for processing a plant having a stem and to a product obtained by the method. The invention can be used advantageously with various plants not necessarily having a stem.

There are methods for processing a plant after harvesting which involve extracting a substance from the plant.

### Problem & Solution

The quality of the substance obtained by some known methods is considered to be insufficient.

It is an object to improve the quality of a desired substance obtainable from the plant.

This object is achieved by the method according to claim 1 (first aspect). Further, a product obtained by the method (second aspect) includes the desired substance of improved quality. Preferred embodiments form the respective subject matter of the dependent claims.

The method according to the first aspect is suitable for processing a plant having a stem. The method includes the steps:
- S3: freezing the plant to a second temperature below -10°C (frozen plant),
- S6: preparing an infusion using the frozen plant and a liquid (cold infusion).

Step S3 provides a frozen plant. For step S6 this frozen plant is used with a liquid, and the step results in an infusion. At the beginning of step S6 the plant is still frozen because of preceding step S3. In combination, steps S3 and S6 can help to gain a mixture or infusion including a desired substance from the plant while the temperatures can be limited during processing. The quality of the desired substance can be improved advantageously by the method, particularly if the desired substance ages faster or suffers otherwise at increased temperatures. The mixture including the liquid and the desired substance as available after step S6 is also called "infusion".

Within the concept of the present invention, the expression "desired substance" can mean a mixture of desired chemical substances and can mean a mixture of chemical substances having a smaller concentration of one or more undesired substances.

### Preferred embodiments

Preferred embodiments explained in the following can be combined with each other, advantageously, unless stated otherwise.

Preferably, cannabis sativa is the plant to be processed by the method. In this case the obtained infusion can have a smaller concentration of cannabidiol (CBD). Alternatively, cannabis indica can be processed by this method. In the obtained infusion, CDB can be present merely at a minimum level or as a trace element.

The method can be performed advantageously on aromatic herbs such as basilica or mint, helping to preserve the flavouring of these plants or herbs.

Preferably, the time interval between harvesting the plant and step S3 is less than 2 hours to preserve the quality of the desired substance as much as possible.

According to a preferred embodiment, the second temperature is between -40°C and -90°C, preferably below -50°C or about -70°C. This can help to preserve the desired substance and improve its quality. Further, crystals can form in the plant during step S3 which can help to weaken or break cell walls of the plant. When the cell walls are weakened, preparing the infusion can be simplified.

In another preferred embodiment, step S3 is performed with a shock freezing apparatus arranged remote from a harvesting site where the plant is harvested. The shock freezing apparatus can be a shock freezing tunnel allowing a continuous processing of the plant during step S3. The shock freezing apparatus can employ CO₂, particularly liquid CO₂. The plant can be frozen more rapidly since the absolute of the temperature gradient |(dT/dt)| is greater. Thereby, the quality of the desired substance can be improved.

A preferred embodiment involves arranging the frozen plant and the liquid in a receptacle during step S6, resulting in a mixture of the plant and the liquid in the receptacle. The frozen plant can be ground (S19) prior to step S6. Drinking water can be the liquid of step S6, which can have a mineralisation of max. 2 g/litre. Alternatively, ethanol can be used for step S6 which ethanol can be evaporated from the infusion and replaced by drinking water (S20) after step S6. This can simplify the processing of the plant. The volume of the liquid can be selected such that the concentration of the desired substance in the infusion after step S6 is twice as high as during subsequent processing of the infusion. This can help to reduce the volume of the infusion or mixture during and after step S6 and to reduce the cost of storage. The volume of liquid can be chosen such that the infusion has a concentration of about 2.5% of the desired substance, after step S6.

Step S6 of a preferred embodiment includes adding heat energy to the liquid or mixture, preferably by a wall section of the receptacle. The receptable can include a stirring device and/or can have a cylindrical section for improved stirring. This can help to extract the desired substance from the plant. When the volume of the liquid is selected such that the concentration of the substance in the infusion after step S6 is twice as high as during subsequent processing of the infusion, then heat energy can be saved.

In a preferred embodiment, the liquid is heated during step S6 to a fourth temperature (T4) above 50°C. The fourth temperature can be between 90°C and 97°C below, preferably about 95°C. The fourth temperature can be held during a first time interval (S6') between 30 and 60 minutes, preferably about 45 minutes. This can help to extract the desired substance from the plant while preserving the quality of the desired substance as much as possible.

It has been found experimentally that subjecting cannabis sativa to step S6 using drinking water as the liquid (and step S3) helps to reduce the concentration of CBD in the later infusion. Further, water soluble substances of cannabis can be gained for the later infusion. The claimed method may help to preserve a unique taste profile of the fresh plant, preserve antioxidants and/or nutritional micro elements.

Alternatively, ethanol is used instead of drinking water in step S6 and cannabis sativa is the plant of steps S3, S6, in which case the infusion can include a greater concentration CBD and/or cannabidiolic acid (CBDA). Further, ethanol soluble substances of cannabis can be gained for the later infusion.

Another preferred embodiment further includes the step of
S13 cooling the infusion to below 25°C (T5) after step S6, preferably to below 19°C.
S13 should be performed very soon after the end of S6, preferably within 30 minutes from the end of step S6. This can improve the yield and/or the quality of the desired substance.

A preferred embodiment comprises steps S3, S6 as well as the following steps:
- S11: removing the plant or plant section from the infusion, particularly after step S6 or S6', preferably with a basket filter, with a fork or by draining the infusion from a receptacle used for step S6 and keeping the plant or plant section in the receptacle,
- S12: filtering the infusion after S11, using a sieve, a paper filter, a centrifuge or another industrial filtering technique,
- S13: cooling the infusion to below 25°C (T5), preferably to below 19°C,
- S14: storing the cooled infusion after S13, particularly in a storage container which can be cooled,
This can help to preserve the quality of the desired substance in the infusion.

Preferably, further liquid can be added to the infusion to dilute it to a desired concentration of the desired substance (S15), when the infusion including the desired substance is taken from the storage container (S18) particularly for subsequent production.

Another preferred embodiment further comprises the step of harvesting the plant (harvested plant) at the harvesting site (step S1). Preferably, step S1 further includes separating the plant's top section including leaves and/or flowers (plant section) from the rest of the harvested plant (S1'), particularly at the harvesting site. Preferably, the plant section obtained by extended step S1 (S1') has a length from 10 to about 35 cm. The plant section can be milled or ground (S19) prior to S6. This can help to reduce the cost and effort for performing the method, since parts of the plant containing less of the desired substance can be sidelined or discarded.

In a preferred embodiment, the time interval between harvesting/step S1 and step S3 is less than 2 hours. This can help to preserve the desired substance in the harvested plant or plant section.

A preferred embodiment further includes the step of cooling or refrigerating the harvested plant or plant section obtained by step S1 to a first temperature below 0°C at the harvesting site (step S2). The first temperature can be between -5°C and -30°C, preferably below -15°C. Step S2 can be performed with a mobile cooling device or refrigerator truck. The time interval between step S2 and step S3 can be less than 2 hours. The time interval between harvesting the plant and step S2 can be less than two hours. This can help to preserve the desired substance in the harvested plant or plant section.

According to a preferred embodiment, the harvested plant or plant section is stored in a perforated container (S16), preferably from plastic. The container can comprise a badge indicating the harvesting site, the point in time of harvesting, the type of plant and/or a lot number. Step S2 can be performed with this perforated container. The perforated container can help to simplify or improve the handling, storing, transporting and/or the freezing the harvested plant or plant section. The harvested plant or plant section can be taken from the perforated container (S17) for performing step S6.

Preferably, the plant of steps S2, S3 and/or S6 is the harvested plant's top section including leaves. The plant section can include flowers. This can help to reduce the cost and effort for performing the method, since parts of the plant containing less of the desired substance can be sidelined or discarded.

A preferred embodiment further includes the step of storing the harvested plant or plant section at a third temperature (T3) below -5°C (S4), preferably between steps S3 and S6. The third temperature can be smaller than the first temperature. The third temperature can be between -10°C and -30°C, preferably below -15°C, preferably about -20°C. This can help to preserve the desired substance in the harvested plant or plant section.

Another preferred embodiment further includes the step of preparing a concentrate from the infusion (S7) obtained by step S6. This embodiment can help to reduce the cost of storing.

A preferred embodiment further includes the step of preparing a food product, a consumer product, a personal care product, an animal food product or the like with the infusion or the concentrate (S8). This can help to provide a product having the desired substance of improved quality.

In a preferred embodiment, 1000 litres of the product are made with 5 kg to 100 kg, preferably about 25 kg, of frozen cannabis, preferably cannabis sativa or cannabis indica. Hemp oil and/or another flavouring can be added to the product.

According to the second aspect, a product is obtained by one of the above methods and embodiments. The product can have the desired substance of improved quality.

1000 litres of a preferred product comprise an infusion from 5 kg to 100 kg, preferably about 25 kg, of cannabis, preferably cannabis sativa or cannabis indica. The preferred product can also comprise hemp oil and/or another flavouring.

### Exemplary embodiments

Further details and advantages of the invention become apparent to the skilled person from the following exemplary embodiments.

The method of a first example includes step S3 and the plant is rapidly frozen to a second temperature (T2) below -10°C, in this example to about -48°C. The second temperature can be in the range of -30°C to -60°C. During step S3, the plant can be held in a perforated plastic container (S16) for simplified processing. The container is routed through a shock freezing tunnel installed remote from the site where the plant was harvested. Freezing of the plant may cause crystals to form in the plant and the cell walls of the plant to weaken. Further, possible aging/degradation of the desired substance of the plant, which can be an essential oil, can be decelerated by the low temperature. The time interval between harvesting the plant and step S3 can be limited not to exceed 2 hours to preserve the quality of the desired substance as much as possible.

The frozen plant is taken from the perforated plastic container (S17) for step S6. The frozen plant can be ground (S19) prior to step S6. During subsequent step S6, an infusion is prepared using the frozen plant and a liquid. In this example drinking water having a mineralisation of max. 2 g/litre is used. The volume of the liquid is chosen such that the concentration of the desired substance in the infusion after step S6 will be twice as high as during subsequent processing of the infusion. The liquid and the frozen plant are arranged in a receptable having a heatable wall section. Heating can be electrical. The receptable can have a cylindrical section and its contents can be stirred during step S6.

Heat energy is added to the mixture including the liquid and the frozen plant, and the temperature of the mixture is controlled to be in the range between 92°C and 97°C (T4). Temperature control of the mixture can be aided by a thermocouple positioned in the liquid and a microcontroller reading the thermocouple's signal and adjusting the heat flux through the receptable wall, e.g. adjusting the electrical heating power. The fourth temperature can be held during a first time interval of about 45 minutes. The heat treatment helps to extract the desired substance from the plant. The temperature and the first time interval are chosen to preserve the quality of the desired substance as much as possible. Afterwards, the infusion includes the plant, the liquid and the desired substance from the plant.

When performing steps S3, S6 on cannabis sativa or cannabis indica, preferably with drinking water as the liquid of step S6, the obtained infusion can have a smaller concentration of cannabidiol (CBD). Alternatively, ethanol can be the liquid of step S6, in which case the concentration of CBD and/or cannabidiolic acid can be increased/enriched in the infusion. The components of the obtained infusion and their concentrations can be determined using chromatography, liquid chromatography or HPLC. HPLC can be performed using an Agilent 1260 HPLC system and a Nucleoshell RP 2.7 µm C8, 150 ^{∗} 4.6 mm column from Macherey-Nagel.

In the first example, a plant section including leaves and/or flowers can be used during steps S3, S6 instead of the entire plant. To this end, the plant's top section including the leaves and/or flowers (plant section) can be separated from the harvested plant prior to step S3. While the plant's top section (plant section) can be kept for subsequent processing (i.e. step S3), the remainder of the plant can be sidelined. The plant section can be stored in a perforated plastic container (S16). This can help to limit the effort of processing the plant, when the desired substance is predominantly included in the leaves or flowers.

In the first example, the harvested plant or separated plant section including leaves can be stored at a temperature (T3) below -5°C, preferably at about -20°C (S4), between steps S3 and S6. This can help to preserve the quality of the desired substance as much as possible. S6 need not follow immediately after S3 because of S4, and S4 can render processing of the plant more flexible.

The method of a second example includes steps S3 and S6 from the first example. Further this method comprises removing the plant from the infusion (S11), preferably with a basket filter, with a fork or by draining the infusion from a receptacle used for step S6 and keeping the plant or plant section in the receptacle, after which the infusion includes the desired substance(s) and the liquid. The infusion is filtered (S12) with a paper filter, a metal filter or a sieve, but a centrifuge is also suitable. The filtered infusion is cooled (S13) to below 19°C. The cooled infusion can be stored (S14) until subsequent processing. It was found that cooling the infusion (S13) contributes significantly to preserving the desired substance in the infusion.

In addition to the steps of the first or second example, the method of a third example comprises cooling or refrigerating the harvested plant or the plant top section including leaves (plant section) to a first temperature (T1) below 0°C, preferably to about -i8°C, at the harvesting site (S2). A refrigerator truck can be used at the harvesting site for step S2. Further, the refrigerator truck can to transport the harvested plant or plant section to the site of the shock freezing apparatus used for step S3. The harvested plant or the plant top section can be arranged in the perforated plastic container (S16) prior and during step S2. Step S2 can help to reduce aging/degradation of the desired substance of the plant.

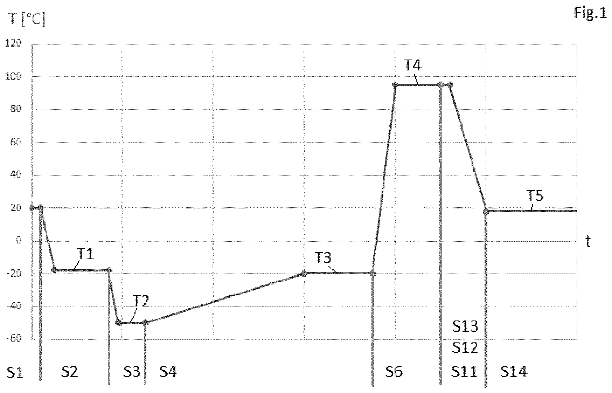

Figure 1 schematically and qualitatively shows a time dependent temperature profile (blue) of an exemplary fourth method and the order of respective steps while some of the temperature gradients (dT/dt) may differ from straight lines in reality. The horizontal axis indicates the time t and the temperature of the plant, mixture or later infusion including the desired substance increases vertically. At point in time t = 0, the plant is harvested (S1) and has ambient temperature. The plant's top section including leaves can be separated very soon after harvesting and can be kept for subsequent processing. The vertical lines (green) indicate begin and/or end of a step and the steps are given between two such vertical lines. Shock freezing (S3) and cold infusion (S6) are compulsory (see the first example) for the exemplary method, while the temperatures may differ from the shown temperature profile. Steps S2, S4, S11 to S14 are optional. Reference is made to the above explanations for steps S1 to S16.

1000 litres of the product are made with 5 kg to 100 kg, preferably about 25 kg, of frozen cannabis, preferably cannabis sativa or cannabis indica. Hemp oil and/or another flavouring can be added to the product.

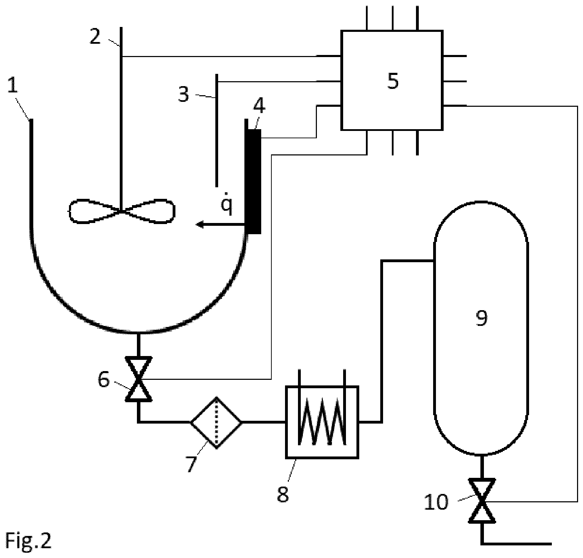

Figure 2 shows an apparatus for performing steps S6, S11, S12, S13 and S14. The apparatus comprises the receptable 1 for accepting the harvested plant or plant section and the liquid. The receptacle has a heated wall section 4 for adding heat energy q to the mixture in the receptacle 2, and a first valve 6 permits to drain the infusion from the receptable. A thermocouple 3 and as stirrer 6 extend into the receptable and mixture. A microcontroller 5 is connected with the stirrer, thermocouple, heated wall section for controlling or regulating the temperature of the mixture in the receptacle. After being released from the receptable, the infusion now including the desired substance passes a filter 7 for removing undesired particles. The filter can be made with paper or metal, can be a sieve or centrifuge. The filtered infusion is cooled by heat exchanger 8, preferably to below 25°C, before being received in a storage container 9. A second valve 10 allows to release a volume of the stored infusion from the storage container 9, which second valve is also controlled by the microcontroller.

## Claims

1. Method for processing a plant having a stem, comprising the following steps:
S3 freezing the plant to a second temperature (T2) below -10°C,
S6 preparing an infusion using the frozen plant and a liquid.

2. Method according to claim 1, wherein the second temperature (T2) is between -40°C and -90°C, preferably below -50°C.

3. Method according to one of the preceding claims, wherein step S3 is performed with a shock freezing apparatus arranged remote from a harvesting site where the plant is harvested.

4. Method according to one of the preceding claims, wherein the frozen plant and the liquid are arranged in a receptacle during step S6.

5. Method according to one of the preceding claims, wherein heat energy is added to the liquid during step S6, preferably by a wall section of the receptacle.

6. Method according to one of the preceding claims, wherein the liquid is heated during step S6 to a fourth temperature (T4) above 50°C.

7. Method according to one of the preceding claims, wherein the liquid of step S6 is drinking water or ethanol.

8. Method according to one of the preceding claims, further comprising the step of
S1 harvesting the plant at the harvesting site (harvested plant).

9. Method according to claim 8, wherein step S1 further includes separating a top section including leaves (plant section) from the harvested plant.

10. Method according to claim 9, wherein the plant section obtained by step S1 has a length from 10 to about 35 cm.

11. Method according to one of claims 8 to 10, wherein the time interval between step S1 and step S3 is less than 2 hours.

12. Method according to one of the preceding claims, further comprising the step of
S2 cooling or refrigerating the harvested plant or the plant section obtained by step Si to a first temperature (T1) below 0°C at the harvesting site.

13. Method according to claim 12, wherein the first temperature (T1) is between -5°C and -30°C, preferably below -15°C.

14. Method according to one of claims 12 to 13, wherein step S2 is performed with a mobile cooling device.

15. Method according to one of claims 12 to 14, wherein step S2 is performed using a perforated container arranged for accepting the plant section obtained by step S1.

16. Method according to one of claims 12 to 15, wherein the time interval between step S2 and step S3 is less than 2 hours.

17. Method according to one of the preceding claims, wherein the plant of steps S2, S3 and/or S6 is the harvested plant's top section including leaves.

18. Method according to one of the preceding claims, further comprising the step of
S4 storing the harvested plant or plant section at a third temperature (T3) below -5°C, preferably before step S6.

19. Method according to claim 18, wherein the third temperature (T3) is between -10°C and -30°C.

20. Method according to one of the preceding claims, comprising
S7 preparing a concentrate from the infusion.

21. Method according to one of the preceding claims, comprising
S8 preparing a food product, a consumer product, a personal care product or an animal food product with the infusion or the concentrate.

22. Product obtained by a method according to one of the preceding claims.
